# EUROPEAN PATENT SPECIFICATION

(11) **EP 4 506 007 B1**
(45) Date of publication and mention of the grant of the patent: **28.10.2026**
(21) Application number: 23785022.7
(22) Date of filing: 06.04.2023
(51) Int. Cl.: A61K 31/575, A61P 11/00, A61P 43/00, A23L 33/10

(54) **COMPOSITION FOR PREVENTING OR TREATING PULMONARY FIBROSIS, COMPRISING TAURODEOXYCHOLIC ACID OR PHARMACEUTICALLY ACCEPTABLE SALT THEREOF AS ACTIVE INGREDIENT**
ZUSAMMENSETZUNG ZUR PRÄVENTION ODER BEHANDLUNG VON LUNGENFIBROSE MIT TAURODEOXYCHOLSÄURE ODER PHARMAZEUTISCH AKZEPTABLEM SALZ DARAUS ALS WIRKSTOFF
COMPOSITION POUR LA PRÉVENTION OU LE TRAITEMENT DE LA FIBROSE PULMONAIRE, COMPRENANT DE L'ACIDE TAURODÉSOXYCHOLIQUE OU UN SEL PHARMACEUTIQUEMENT ACCEPTABLE DE CELUI-CI EN TANT QUE PRINCIPE ACTIF

(30) Priority: 06.04.2022 KR 20220042665
(43) Date of publication of application: 12.02.2025
(73) Proprietor: Shaperon Inc., Seoul 06373 (KR)
(72) Inventor: SEONG, Seung Yong, Seoul 05507 (KR); HWANG, Jeong Jung, Seoul 06370 (KR)
(74) Representative: Vossius & Partner Patentanwälte Rechtsanwälte mbB
(86) International application number: PCT/KR2023/004665
(87) International publication number: WO 2023/195798

(56) References cited:
- WO-A1-2006/134022
- WO-A1-2011/157655
- WO-A1-2020/176703
- KR-A- 20150 024 805
- US-A1- 2017 196 895
- US-B2- 10 071 107
- HAAK ANDREW J ET AL: "Targeting GPCR Signaling for Idiopathic Pulmonary Fibrosis Therapies", TRENDS IN PHARMACOLOGICAL SCIENCES, ELSEVIER, HAYWARTH, GB, vol. 41, no. 3, 30 January 2020 (2020-01-30), pages 172 - 182, XP086060780, ISSN: 0165-6147, [retrieved on 20200130], DOI: 10.1016/J.TIPS.2019.12.008
- PAOLO COMEGLIO ET AL: "Beneficial effects of bile acid receptor agonists in pulmonary disease models", EXPERT OPINION ON INVESTIGATIONAL DRUGS, vol. 26, no. 11, 3 October 2017 (2017-10-03), UK, pages 1215 - 1228, XP055676851, ISSN: 1354-3784, DOI: 10.1080/13543784.2017.1385760
- SAITO SHIGEKI ET AL: "Pharmacotherapy and adjunctive treatment for idiopathic pulmonary fibrosis (IPF)", JOURNAL OF THORACIC DISEASE, vol. 11, no. S14, 1 September 2019 (2019-09-01), China, pages S1740 - S1754, XP093226860, ISSN: 2072-1439, DOI: 10.21037/jtd.2019.04.62
- BIN TONG;LIN FU;BIAO HU;ZHI-CHENG ZHANG;ZHU-XIA TAN;SE-RUO LI;YUAN-HUA CHEN;CHENG ZHANG;HUA WANG;DE-XIANG XU;HUI ZHAO: "Tauroursodeoxycholic acid alleviates pulmonary endoplasmic reticulum stress and epithelial-mesenchymal transition in bleomycin-induced lung fibrosis", BMC PULMONARY MEDICINE, vol. 21, no. 1, 5 May 2021 (2021-05-05), London, UK , pages 1 - 11, XP021290437, DOI: 10.1186/s12890-021-01514-6
- HORVATITS THOMAS, DROLZ ANDREAS, RUTTER KAROLINE, ROEDL KEVIN, LANGOUCHE LIES, VAN DEN BERGHE GREET, FAULER GÜNTER, MEYER BRIGITTE: "Circulating bile acids predict outcome in critically ill patients", ANNALS OF INTENSIVE CARE, vol. 7, no. 1, 1 December 2017 (2017-12-01), pages 1 - 10, XP093097916, DOI: 10.1186/s13613-017-0272-7

## Description

### [Technical Field]

The present invention relates to a composition for the prevention or treatment of pulmonary fibrosis, comprising taurodeoxycholic acid or a pharmaceutically acceptable salt thereof as an active ingredient.

### [Background Art]

The cause of inflammation is known as PAMPs (pathogenassociated molecular patterns) caused by pathogen infection and DAMPs (damage-associated molecular patterns) caused by noninfection damage in tissues, and the failure to control the protective or inflammatory response in vivo involving immune cells, blood vessels, and inflammatory mediators is known to cause inflammatory diseases. Meanwhile, steroids, NSAIDs (non-steroidal anti-inflammatory drugs), antibody treatments, and JAK inhibitors are currently being prescribed as treatments for inflammatory diseases, but steroid drugs remain in short-term prescriptions due to systemic side effects, and NSAIDs are not showing sufficient efficacy in the clinical stage. In addition, antibody treatments can be given relatively quickly and administered for a long time, but there are side effects at the injection site, a gradual decrease in efficacy occurs due to the production of neutralizing antibodies in the body, and the patient burden is high. Meanwhile, JAK inhibitors have excellent anti-inflammatory efficacy but have been at risk of increasing severe infections, and cardiovascular side effects and carcinogenic risks have been confirmed in clinical trials. Intractable inflammatory diseases have a high recurrence rate and require long-term administration for treatment. However, treatments developed so far do not meet these unmet market demands, and therefore, the development of new treatments is necessary.

The formation of the NLRP3 inflammasome, which plays a central role in the inflammatory response within the body, is a key component of the innate immune system that mediates the activation of caspase-1 and the secretion of pro-inflammatory cytokines IL-1β and IL-18 in response to microbial infection and cellular damage. The inflammasome is a cytosolic multiprotein oligomer of the innate immune system responsible for the activation of inflammatory responses. Activation of the inflammasome promotes not only the cleavage of gasdermin-D through caspase-1, but also the proteolytic processing, maturation, and secretion of pro-inflammatory cytokines pro-IL-1β and pro-IL-18 into their active forms IL-1β and IL-18, respectively. Dysregulation of inflammasome activation may lead to various diseases, including cancer, metabolic disorders, degenerative diseases, and inflammatory diseases. Specifically, abnormal activation of the NLRP3 inflammasome is associated with the onset and exacerbation of various inflammatory diseases such as ulcerative colitis, gout, multiple sclerosis, arthritis, sepsis, and neuroinflammatory disorders. The NLRP3 inflammasome is also activated by pathogenassociated molecular patterns (PAMPs) from pathogens such as viruses and bacteria, and by danger-associated molecular patterns (DAMPs), such as calcium influx, mitochondrial reactive oxygen species, and extracellular ATP.

Meanwhile, the P2X7 receptor is an ion channel receptor composed of a complex belonging to the P2Xn purinergic receptor family. Stimulation of P2X7 promotes the extracellular release of potassium ions and facilitates the rapid influx of calcium and sodium ions into the cell. In disease states caused by the occurrence of inflammatory factors inside and outside the body, the concentration of extracellular ATP significantly increases, activating the P2X7 receptor. This activation triggers the efflux of potassium ions and intracellular ionic imbalance, which promotes the NLRP3 inflammasome. Furthermore, activation of the NLRP3 inflammasome leads to the activation of pro-IL-1β and pro-IL-18, which in turn promotes cell death and exacerbates the inflammatory response in surrounding tissues. The P2X7 receptor, which plays a critical role in the progression of the inflammatory response, is widely expressed in various immune cells, epithelial cells, and neurons. Since P2X7 receptor signaling activates both canonical and non-canonical pathways of NLRP3 activation, the ATP-P2X7 interaction is crucial for the activation of the NLRP3 inflammasome. For example, high concentrations of ATP released from necrotic cells act as pro-inflammatory DAMPs that bind to the P2X7 receptor and activate the NLRP3 inflammasome.

Attempts to develop new drugs targeting the NLRP3 inflammasome, a key regulator of the inflammatory response, have been made multiple times in the past. In recent years, it has been discovered that the inflammasome is activated through both canonical and non-canonical pathways involving pro-caspase, gasdermin D, ASC, NLR proteins, AIM2, IFI16, pyrin, and others. Moreover, it has become known that dysregulation of the inflammasome in these processes leads to various diseases. As a result, since 2010, the development of drugs targeting proteins involved in inflammasome-related signaling has been actively pursued. Representative inflammasome modulators that have been developed include P2X7 receptor antagonists, NLRP3 inhibitors, and caspase-1 inhibitors. Specifically, P2X7 antagonists were developed by global pharmaceutical companies such as Pfizer, AstraZeneca, and Janssen, with indications for rheumatoid arthritis, Crohn's disease, and depression. However, most of these development efforts were discontinued due to insufficient efficacy observed in clinical trials. The development of caspase-1 inhibitors was halted in 2020 due to failure to meet efficacy endpoints and safety concerns in clinical trials, leaving no global development pipeline for this class of drugs. NLRP3 inhibitors are being developed by venture companies such as OLATEC, IMFLAZOME, IFM THERAPEUTICS, NODTHERA, and AC Immune, with indications for osteoarthritis, systolic heart failure, Parkinson's disease, inflammatory bowel disease, and Alzheimer's disease. As previously discussed, most of these inflammasome modulators in drug development have not achieved success in clinical trials. The underlying reason is that these agents, targeting only specific drug targets, are unable to effectively inhibit the diversity of pro-inflammatory pathways that activate the NLRP3 inflammasome. Specifically, the currently known inflammasome modulators, including P2X7 receptor antagonists, NLRP3 inhibitors, and caspase-1 inhibitors, selectively inhibit inflammatory cytokines triggered by inflammation, such as IL-1β and IL-18, but they do not inhibit TNF-α, an inflammatory cytokine that is activated at the onset of inflammation. This represents a fundamental limitation of drugs that selectively target only NLRP3 or P2X7, as pharmacological mechanisms that selectively block only the inflammatory activation phase are insufficient to suppress the inflammatory response via various bypass pathways. Consequently, there have been numerous reports in clinical settings of unmet patient drug responsiveness and anti-inflammatory effects concerning the current inflammasome modulators due to the compensatory activation of alternative bypass pathways.

Additionally, due to the genetic redundancy and polymorphism inherent in NLRP3 and P2X7, it has been revealed that the current inflammasome modulators do not yield the targeted therapeutic response rates in actual clinical settings. Specifically, because they exist in a homologous NLRP inflammasome family that possesses the same protein domains as the NLRP3 inflammasome, this has been identified as a cause of off-target side effects of the inflammasome modulators. The P2X7 and NLRP3 proteins are associated with hundreds to thousands of single nucleotide polymorphisms (SNPs), leading to high genetic diversity among individuals. This results in significant interindividual variability in drug response rates and efficacy, suggesting that new drugs targeting these proteins are unlikely to achieve broad therapeutic efficacy without personal variance. As discussed above, the currently developed inflammasome-targeted drugs, including P2X7 receptor antagonists, NLRP3 inhibitors, and caspase-1 inhibitors, exhibit numerous limitations in achieving the intended clinical efficacy.

As an attempt to overcome the limitations of existing inflammasome-targeted drugs, recent efforts are focused on developing therapeutics that target GPCR19, which regulates upstream signaling of the inflammasome. Specifically, TUDCA, INT-747 (Obeticholic acid, OCA), and INT-777 (S-EMCA) are representative examples. TUDCA is currently undergoing clinical trials for conditions such as type 2 diabetes, ulcerative colitis, and amyotrophic lateral sclerosis (ALS). Likewise, INT-747 is also being tested in clinical trials for diseases including biliary cirrhosis, non-alcoholic steatohepatitis, and cirrhosis. All of these compounds are bile acid derivatives and are under investigation as agonists of GPCR19. In particular, GPCR19 is predominantly expressed in immune cells, selectively inducing immune cell-mediated anti-inflammatory effects while minimizing nonspecific side effects on normal cells. This contrasts with existing anti-inflammatory agents, such as steroids and JAK inhibitors, whose drug targets are distributed systemically beyond the site of inflammation. Consequently, these agents induce broad systemic responses, leading to reported side effects such as systemic metabolic disturbances, increased risk of infections, and heightened carcinogenic risks. Given these considerations, GPCR19-targeted therapeutics are anticipated to have significant advantages in terms of safety.

To date, there are no GPCR19-targeted therapeutics that have demonstrated efficacy in clinical trials. One of the most advanced GPCR19-targeted therapies encountered a failure in Phase 3 clinical trials aimed at inflammatory diseases due to insufficient anti-inflammatory effects. In fact, the initial GPCR19-targeted therapeutics were derived using artificially overexpressed cell lines without a thorough investigation of the anti-inflammatory mechanisms of GPCR19. These drugs were developed through systems that did not accurately reflect the initiation and activation of inflammation via GPCR19 in vivo, leading to clinical failure cases. Therefore, there remains a critical need for the development of new drugs with broad anti-inflammatory effects that can effectively block both the initiation and activation of inflammation through GPCR19-targeted therapy.

Recently, bile acids have emerged as novel compounds being studied as regulators of GPCR19. Bile acids are amphipathic molecules that possess both hydrophobic and hydrophilic regions. Examining the biosynthesis of bile acids, cholesterol serves as a precursor that is converted in the liver by cytochrome P450 enzymes into primary bile acids, specifically cholic acid and chenodeoxycholic acid. Subsequently, these primary bile acids are conjugated with taurine or glycine by hepatocytes, enhancing their hydrophilicity as they move into the intestine, where they are transformed into secondary bile acids by gut microbiota. Secondary bile acids are reabsorbed into the liver through the enterohepatic circulation and travel via the bloodstream to various tissues, where they perform a range of physiological functions. Specifically, they facilitate the digestion of fats through their amphipathic properties, inhibit the overgrowth of harmful gut microorganisms through their acidity, and promote the excretion of cholesterol during the enterohepatic circulation process. Additionally, it is known that secondary bile acids transmit signals through receptors such as the farnesoid X receptor (FXR), which is a nuclear receptor, and G protein-coupled bile acid receptor 1 (GPBAR1, GPCR19), which is a G protein-coupled receptor located in the cell membrane. Specifically, bile acids bound to the farnesoid X receptor inhibit gluconeogenesis and lipogenesis genes, thereby suppressing gluconeogenesis and lipogenesis in the liver. The interaction with G protein-coupled bile acid receptor 1 promotes metabolic activity, enhances insulin secretion, and suppresses cytokine release. Notably, the binding to G protein-coupled bile acid receptor 1 also inhibits inflammation response, which has led to active research on the treatment of inflammatory and autoimmune diseases related to bile acids in conditions affecting the skin, nervous system, stomach, intestines, liver, and joints.

Fibrosis can be defined as the abnormal or excessive accumulation of extracellular matrix, particularly fibrillar collagen, referring to the abnormal accumulation of collagen matrix due to injury or inflammation that alters the structure and function of various tissues. Inflammation is central to the development of fibrosis in the lungs, and inflammatory cells, including mast cells, are abundantly found in pulmonary fibrosis.

Pulmonary fibrosis is a chronic disease that causes swelling and scarring of the alveoli and interstitial tissue of the lungs. This scar tissue replaces healthy tissue, leading to inflammation, and chronic inflammation can be identified as a precursor to fibrosis. Such damage to lung tissue may result in stiffness of the lungs, making it difficult for the individual to breathe autonomously.

Idiopathic Pulmonary Fibrosis (IPF) is defined as a specific form of chronic, progressive, irreversible fibrosing interstitial pneumonia of unknown origin, primarily occurring in the elderly. In the United States, 100,000 individuals suffer from idiopathic pulmonary fibrosis, with a high risk of death within a few years of diagnosis and a poor prognosis. Treatment options are limited to halting disease progression and providing supplemental oxygen to improve symptoms and lung function. The clinical course of IPF often includes acute exacerbations that lead to hospitalization.

In patients with idiopathic pulmonary fibrosis, antifibrotic drugs such as pirfenidone and nintedanib are used. The pharmacological mechanism of these drugs is as follows: pirfenidone exhibits antifibrotic effects by inhibiting the proliferation of fibroblasts and collagen synthesis, while nintedanib is a low molecular weight tyrosine kinase inhibitor that targets fibroblast growth factor receptors. The main target receptors are platelet-derived growth factor receptors (PDGFRα, β), fibroblast growth factor receptors (FGFR1, 2, 3), and vascular endothelial growth factor receptors (VEGFR1, 2, 3). It has a pathological mechanism that competitively binds to target receptors and blocks the intracellular signaling processes. These drugs have been proven to have the effect of delaying the progression of the disease; however, they do not fundamentally treat the condition. Specifically, in the case of pirfenidone, over 10% of users experience side effects related to the gastrointestinal system (such as indigestion, loss of appetite, nausea, diarrhea), fatigue, headache, and rash. Additionally, 1-10% of users report central nervous system side effects (such as insomnia, dizziness, drowsiness), skin issues (such as photosensitivity, pruritus), gastrointestinal problems (such as taste abnormalities, abdominal pain, abdominal distension), weight loss, elevated liver enzymes level, chest pain, joint pain, and weakness. Less than 1% of users have reported side effects such as angioedema and agranulocytosis. For nintedanib, over 10% of users exhibit gastrointestinal abnormalities (such as diarrhea, vomiting, nausea, abdominal pain), hepatobiliary abnormalities (such as elevated liver enzymes, increased alanine aminotransferase (ALT)), metabolic and nutritional issues, and decreased appetite. Furthermore, 1-10% of users experience hepatobiliary abnormalities (such as drug-induced liver injury, increased aspartate aminotransferase (AST), elevated alkaline phosphatase (ALKP), and increased gamma-glutamyl transferase (GGT)), vascular issues (such as hypertension and bleeding), metabolic and nutritional problems (such as weight loss), and neurological issues (such as headache). Side effects, reported in less than 1% of users, include gastrointestinal issues (such as pancreatitis), hepatobiliary abnormalities (such as hyperbilirubinemia), hematologic and lymphatic disorders (such as thrombocytopenia), and skin and subcutaneous tissue disorders (such as alopecia). Additionally, currently under development as treatments for idiopathic pulmonary fibrosis are drugs such as pentraxin-2, connective tissue growth factor (CTGF) inhibitors, autotaxin inhibitors, PI3 kinase/mTOR inhibitors, and galectin-3 inhibitors. Many clinical studies are being conducted; however, there is still no drug available that can cure this disease, and patients with advanced disease may need to consider lung transplantation in some cases. Therefore, there is an urgent need for the development of therapeutic agents that can show treatment effects for idiopathic pulmonary fibrosis.

Moreover, the pharmaceutical compositions prepared for the treatment of idiopathic pulmonary fibrosis, including these conventional techniques, provide only partial therapeutic effects or offer negligible levels of efficacy, resulting in a lack of significance and failing to provide fundamental effects that can delay or treat the onset or progression of pulmonary fibrosis.

Furthermore, the pharmaceutical compositions developed for the treatment of idiopathic pulmonary fibrosis, including those based on conventional techniques, exhibit low biocompatibility due to their chemical composition, have a high incidence of side effects, and face challenges in large-scale production and quality control when considering productivity.

Tong, B., Fu, L., Hu, B. et al., BMC Pulm Med 21, 149 (2021), discloses the effects of the bile acid Tauroursodeoxycholic acid (TUDCA) for the treatment of lung fibrosis.

In this regard, the present inventors have made efforts to develop a therapeutic agent that demonstrates excellent therapeutic effects in pulmonary fibrosis while being safe and having minimal side effects. As a result, they confirmed that the administration of a pharmaceutically acceptable salt of taurodeoxycholic acid reduces pulmonary fibrosis and the expression of pulmonary fibrosis-related factors in an animal model of idiopathic pulmonary fibrosis. By revealing that taurodeoxycholic acid or its pharmaceutically acceptable salt can be utilized as an active ingredient in a composition for the prevention or treatment of pulmonary fibrosis, this application has been made.

### [Prior Art Documents]

### [Patent Documents]

WO 2019-139956 (2019.07.18)
WO 2015-189401(2015.12.17)
WO 2017-152048 (2017.09.08)
WO 2019-126089 (2019.06.27)

### [Non-Patent literature]

Won-Il Choi, Current and future treatment for idiopathic pulmonary fibrosis, J Korean Med Assoc. 2021;64(4):256-263

### [Detailed Description of the Invention]

### [Technical Problem]

The objective of the present invention is to provide a composition for the prevention, treatment, or improvement of pulmonary fibrosis that has excellent efficacy and minimal side effects, comprising taurodeoxycholic acid or a pharmaceutically acceptable salt thereof as an active ingredient.

### [Solution to Problem]

To achieve the objectives of the present invention, the present invention provides a pharmaceutical composition for the prevention or treatment of pulmonary fibrosis, comprising taurodeoxycholic acid or a pharmaceutically acceptable salt thereof as an active ingredient.

In addition, the present invention provides a health functional food composition for the prevention or improvement of pulmonary fibrosis, comprising taurodeoxycholic acid or a pharmaceutically acceptable salt thereof as an active ingredient.

In addition, the present invention provides a food composition for the prevention or improvement of pulmonary fibrosis, comprising taurodeoxycholic acid or a pharmaceutically acceptable salt thereof as an active ingredient.

In addition, the present invention provides the use of taurodeoxycholic acid or a pharmaceutically acceptable salt thereof as a pharmaceutical composition for the prevention or treatment of pulmonary fibrosis.

In addition, the present invention provides the use of taurodeoxycholic acid or a pharmaceutically acceptable salt thereof as a health functional food composition for the prevention or improvement of pulmonary fibrosis.

In addition, the present invention provides the use of taurodeoxycholic acid or a pharmaceutically acceptable salt thereof for the preparation of a pharmaceutical composition for the prevention or treatment of pulmonary fibrosis.

Furthermore, the present invention provides the use of taurodeoxycholic acid or a pharmaceutically acceptable salt thereof for the preparation of a health functional food composition for the prevention or improvement of pulmonary fibrosis.

### [Advantageous Effects of Invention]

The inventors have confirmed that administration of a pharmaceutically acceptable salt of taurodeoxycholic acid to an idiopathic pulmonary fibrosis animal model induced by bleomycin and a pulmonary fibrosis cell model induced by TGF-β results in a reduction of pulmonary fibrosis and the expression of pulmonary fibrosis-related factors. Therefore, taurodeoxycholic acid or a pharmaceutically acceptable salt thereof can be utilized as an active ingredient in a composition for the prevention or treatment of pulmonary fibrosis.

### [Brief Description of Drawings]

Fig. 1 illustrates a method for creating an animal model of idiopathic pulmonary fibrosis induced by bleomycin, and administering sodium taurodeoxycholate (hereinafter referred to as "HY209"), a sodium salt of taurodeoxycholic acid, to the aforementioned animal model.
Fig. 2 shows the changes in body weight observed after the administration of HY209 in an animal model of idiopathic pulmonary fibrosis induced by bleomycin.
Fig. 3 illustrates the changes in lung weight observed after the administration of HY209 in an animal model of idiopathic pulmonary fibrosis induced by bleomycin (*p<0.05 compared to the natrosol treatment group, #p<0.05 compared to the pulmonary fibrosis group).
Fig. 4 illustrates the histopathological changes observed after the administration of HY209 in an animal model of idiopathic pulmonary fibrosis induced by bleomycin (#p<0.05 compared to the control group, *p<0.05 compared to the natrosol treatment group, **p<0.05 compared to the pulmonary fibrosis group).
Fig. 5 illustrates the changes in the distribution of α-SMA expression after the administration of HY209 in an animal model of idiopathic pulmonary fibrosis induced by bleomycin (*p<0.05 compared to the natrosol treatment group, #p<0.05 compared to the pulmonary fibrosis group).
Fig. 6 illustrates the changes in the expression of Col1α1, α-SMA, or PAI-1 after inducing fibrosis in the human lung fibroblast cell line MRC5 with TGF-β and simultaneously treating with either HY209 or TUDCA (*p<0.05 compared to the TGF-β only treatment group; **p<0.01 compared to the TGF-β only treatment group) .
Fig. 7a illustrates the differentiation of the human monocytic cell line U937 into macrophages using PMA (phorbol 12-myristate 13-acetate) and the subsequent induction of an inflammatory response with BzATP (2'(3')-O-(4-Benzoylbenzoyl)adenosine 5'-triphosphate), measuring the inhibitory efficacy of HY209 or TUDCA on the production of the inflammatory cytokine IL-1β.
Fig. 7b illustrates the differentiation of the U937 cell line into macrophages using PMA (phorbol 12-myristate 13-acetate) and the subsequent induction of an inflammatory response with Pam2CSK4 (Pam2CysSerLys4), measuring the inhibitory efficacy of HY209 or TUDCA on the production of the inflammatory cytokine TNF-α.

### [Best Mode for Carrying Out the Invention]

Hereinafter, the present invention will be described in more detail.

In the present invention, the term "prevention" refers to any act of inhibiting or delaying the occurrence, spread, and recurrence of a disease through the administration of the composition of the present invention, while the term "treatment" refers to any act of improving or beneficially altering the symptoms of a disease through the administration of the composition of the present invention.

In the present invention, the term "administration" refers to the provision of a predetermined substance to a patient by any suitable method, and the administration route of the composition of the present invention may be via oral or non-oral administration through any general route that can reach the target tissue. Additionally, the composition may be administered by any device that allows the active substance to move to the target cells.

The present invention provides a pharmaceutical composition for the prevention or treatment of pulmonary fibrosis, comprising taurodeoxycholic acid or a pharmaceutically acceptable salt thereof as an active ingredient.

In addition, the present invention provides the use of taurodeoxycholic acid or a pharmaceutically acceptable salt thereof as a pharmaceutical composition for the prevention or treatment of pulmonary fibrosis.

In addition, the present invention provides the use of taurodeoxycholic acid or a pharmaceutically acceptable salt thereof for the preparation of a pharmaceutical composition for the prevention or treatment of pulmonary fibrosis.

In the present invention, the taurodeoxycholic acid is a type of bile acid in the form of taurine-conjugated deoxycholic acid, having the chemical structure described in [Chemical Formula 1] below, and more specifically having the chemical structure described in [Chemical Formula 2] below:

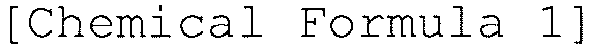

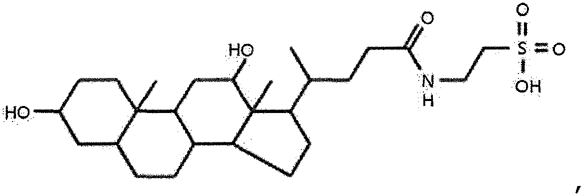

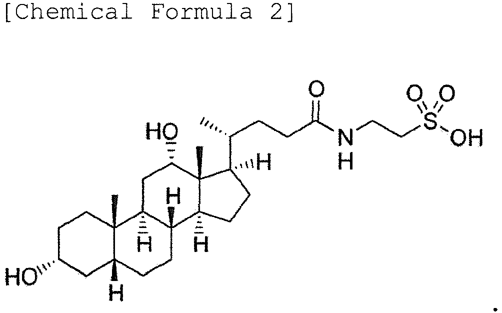

Furthermore, the taurodeoxycholic acid may be used regardless of whether it is isolated from animal carcasses, such as those of cattle, pigs, sheep, dogs, goats, or rabbits, obtained commercially, or synthesized.

In the present invention, pulmonary fibrosis refers to the formation or development of excessive fibrous connective tissue in the lungs (fibrosis), which indicates the progression of scarred (fibrotic) tissue. Specifically, pulmonary fibrosis is a chronic disease that causes swelling and scarring of the alveolar and interstitial tissues of the lungs. This scar tissue replaces healthy tissue, leading to inflammation, and chronic inflammation can be seen as a precursor to fibrosis. Damage to lung tissue of this nature can result in stiffness in the lungs, making it difficult for the individual to breathe independently.

Additionally, pulmonary fibrosis may specifically refer to idiopathic pulmonary fibrosis, nonspecific interstitial pneumonia, acute interstitial neumonia, cryptogenic organizing pneumonia, respiratory bronchiolitis-associated interstitial lung disease, desquamative interstitial pneumonia, lymphoid interstitial pneumonia, interstitial pulmonary fibrosis, or diffuse pulmonary fibrosis, and may more specifically refer to idiopathic pulmonary fibrosis, but is not limited to this.

The aforementioned pulmonary fibrosis can develop due to various causes, such as micro-damage to the lungs induced by the inhalation of fine particles (including asbestos, talc, metal dust, particles present in cigarette smoke, silica dust, etc.). Additionally, pulmonary fibrosis may arise as a secondary effect of other diseases (such as autoimmune diseases, viral or bacterial infections) and can be induced by specific medications, including cytotoxic agents (such as bleomycin, busulfan, and methotrexate), antibiotics (such as nitrofurantoin and sulfasalazine), antiarrhythmic drugs (such as amiodarone and tocainide), anti-inflammatory drugs (such as gold and penicillamine), and illicit drugs (such as narcotics, cocaine, and heroin). In the case of idiopathic pulmonary fibrosis, it may manifest due to unknown causes other than the aforementioned ones.

In the present invention, the composition can prevent or treat pulmonary fibrosis by reducing the expression of pulmonary fibrosis-related factors, such as Col1α1, α-SMA, or PAI-1.

In a specific embodiment of the present invention, the inventors administered a pharmaceutically acceptable salt of taurodeoxycholic acid to a bleomycin-induced idiopathic pulmonary fibrosis animal model and confirmed a decrease in pulmonary fibrosis and the expression of the pulmonary fibrosis-related factor α-SMA. Furthermore, in a cell model of pulmonary fibrosis induced by TGF-β, administering a pharmaceutically acceptable salt of taurodeoxycholic acid resulted in a decrease in the expression of pulmonary fibrosis-related factors Col1α1, α-SMA, and PAI-1. Accordingly, the taurodeoxycholic acid or its pharmaceutically acceptable salt can be utilized as an active ingredient in pharmaceutical compositions for the prevention or treatment of pulmonary fibrosis.

The taurodeoxycholic acid of the present invention encompasses pharmaceutically acceptable salts, solvates, hydrates, racemates, or stereoisomers that can be prepared therefrom.

The taurodeoxycholic acid of the present invention can be used in the form of a pharmaceutically acceptable salt, wherein the salts formed from a pharmaceutically acceptable free acid are useful. The acid addition salts are obtained from inorganic acids such as hydrochloric acid, nitric acid, phosphoric acid, sulfuric acid, hydrobromic acid, hydroiodic acid, nitrous acid, or phosphorous acid, and from non-toxic organic acids such as aliphatic mono- and dicarboxylates, phenyl-substituted alkanoates, hydroxy alkanotes, and alkanedioates, aromatic acids, and aliphatic and aromatic sulfonic acids. The pharmaceutically acceptable non-toxic salts include sulfates, pyrosulfates, bisulfates, sulfites, bisulfites, nitrates, phosphates, monohydrogen phosphates, dihydrogen phosphates, metaphosphates, pyrophosphates, chlorides, bromides, iodides, fluorides, acetates, propionates, decanoates, caprylates, acrylates, formates, isobutyrates, caprates, heptanoates, propiolates, oxalates, malonates, succinates, suberates, sebacates, fumarates, maleates, butin-1,4-dioates, hexane-1,6-dioates, benzoates, chlorobenzoates, methylbenzoates, dinitrobenzoates, hydroxybenzoates, methoxybenzoates, phthalates, terephthalates, benzenesulfonates, toluenesulfonates, chlorobenzenesulfonates, xylensulfonates, phenylacetates, phenylpropionates, phenylbutyrates, citrates, lactates, hydroxybutyrates, glycolates, malates, tartrates, methanesulfonates, propanesulfonates, naphthalene-1-sulfonates, naphthalene-2-sulfonates, or mandelates.

The acid addition salts according to the present invention can be prepared by conventional methods. For example, the taurodeoxycholic acid of the present invention is dissolved in an excess of an aqueous acid solution, and the resulting salt is precipitated using a water-miscible organic solvent, such as methanol, ethanol, acetone, or acetonitrile. Additionally, the mixture can be dried by evaporating the solvent or excess acid, or the precipitated salt can be collected by suction filtration.

Pharmaceutically acceptable metal salts can also be prepared by using a base. Alkali metal or alkaline earth metal salts can be obtained, for example, by dissolving the compound in an excess of an alkali metal hydroxide or alkaline earth metal hydroxide solution, filtering the insoluble compound salt, and evaporating and drying the filtrate. In this case, it is pharmaceutically suitable to prepare sodium, potassium, or calcium salts, and more specifically, it is suitable to prepare the sodium salt in the case of taurodeoxycholic acid. Additionally, the corresponding silver salt can be obtained by reacting the alkali metal or alkaline earth metal salt with an appropriate silver salt (e.g., silver nitrate).

The taurodeoxycholic acid of the present invention, or its pharmaceutically acceptable salts, can be included in a therapeutically effective amount and may comprise a pharmaceutically acceptable carrier.

As used in this specification, "effective amount" refers to an amount of the compound of the present invention sufficient to delay or minimize a disease; or to provide a therapeutic benefit in the treatment or management of a disease.

The pharmaceutically acceptable carriers can include, for example, carriers for oral or parenteral administration. Carriers for oral administration may include lactose, starch, cellulose derivatives, magnesium stearate, stearic acid, and the like. Additionally, carriers for parenteral administration may include water, suitable oils, saline, aqueous glucose, glycols, and the like and may further include stabilizers and preservatives. Suitable stabilizers include antioxidants such as sodium bisulfite, sodium sulfite, or ascorbic acid. Suitable preservatives include benzalkonium chloride, methyl- or propyl-paraben, and chlorobutanol. Other pharmaceutically acceptable carriers can be referred to in the following literature. (Remington's Pharmaceutical Sciences, 19th ed., Mack Publishing Company, Easton, PA, 1995).

The pharmaceutical composition of the present invention can be administered by any route to mammals, including humans. It can be administered orally or parenterally.

The parenteral administration method may be, for example, intravenous, intramuscular, arterial, intracerebral, intrathecal, intracardiac, percutaneous, subcutaneous, intraperitoneal, intranasal, intestinal, topical, sublingual, or rectal administration, but is not limited thereto. For example, the pharmaceutical composition of the present invention may be prepared in an injection-type formulation, and may be administered by a method of lightly pricking the skin with a thin 30-gauge injection needle, or by directly applying or adhering the composition to the skin.

The pharmaceutical composition of the present invention can be formulated into formulations for oral administration or parenteral administration according to the administration route as described above.

In the case of oral administration formulations, the composition of the present invention may be formulated using methods known in the art, such as powder, granule, tablet, pills, sugar tablet, capsule, liquid, gel, syrup, slurry, suspension, etc. For example, oral formulations can obtain tablets by blending the active ingredients with solid excipients, then crushing them, adding suitable supplements, and processing them into a granular mixture. Examples of suitable excipients may include sugars including lactose, dextrose, sucrose, sorbitol, mannitol, xylitol, erythritol and maltitol, starch including corn starch, wheat starch, rice starch and potato starch, cellulose including cellulose, methyl cellulose, sodium carboxymethylcellulose and hydroxypropylmethyl-cellulose, and fillers such as gelatin and polyvinylpyrrolidone. Also, in some cases, crosslinked polyvinylpyrrolidone, agar, alginic acid, sodium alginate, or the like may be added as a disintegrant. Furthermore, the pharmaceutical composition of the present invention may further include an anticoagulant, a lubricant, a wetting agent, a flavoring agent, an emulsifier, and a preservative.

In the case of parenteral administration formulations, they can be formulated by methods known in the art in the form of injections, creams, lotions, external ointments, oils, moisturizers, gels, patches, aerosols, and nasal aspirants. These formulations are described in the prescription literature commonly known in all pharmaceutical chemistry (Remington's Pharmaceutical Science, 15th Edition, 1975. Mack Publishing Company, Easton, Pennsylvania 18042, Chapter 87: Blaug, Seymour).

The total dose of the pharmaceutical composition of the present invention can be administered to a patient as a single dose, or it can be administered to a patient as multiple doses through a fractionated treatment protocol. The pharmaceutical composition of the present invention can vary the amount of active ingredients according to the symptoms of the disease. Specifically, the total dosage of the composition of the present invention may range from 0.01 µg to 1,000 mg per 1 kg of patient body weight per day, more specifically from 0.1 µg to 100 mg. However, the dose of the pharmaceutical composition of the present invention will allow those skilled in the art to determine an appropriate effective dose, taking into account various factors such as the patient's age, weight, health status, gender, disease severity, diet, and excretion rate, as well as the route and number of treatments. The pharmaceutical composition according to the present invention is not specifically limited to any particular formulation, route of administration, or method of administration as long as it exhibits the effects of the present invention.

Furthermore, the pharmaceutical composition of the present invention can be administered as a monotherapy or in combination with other therapeutic agents. When administered in combination with other therapeutic agents, the composition of the present invention and the other therapeutic agents can be administered concurrently, individually, or sequentially. In this case, the other therapeutic agent can be a substance already known to have treatment or improvement effects of pulmonary fibrosis. When the pharmaceutical composition of the present invention is administered in combination with other therapeutic agents, the composition of the present invention and other therapeutic agents can be separately formulated in separate containers, or can be complex formulated together in the same formulation.

In addition, the present invention provides a health functional food composition for the prevention or improvement of pulmonary fibrosis, comprising taurodeoxycholic acid or a pharmaceutically acceptable salt thereof as an active ingredient.

In addition, the present invention provides the use of taurodeoxycholic acid or a pharmaceutically acceptable salt thereof as a health functional food composition for the prevention or improvement of pulmonary fibrosis.

Furthermore, the present invention provides the use of taurodeoxycholic acid or a pharmaceutically acceptable salt thereof for the preparation of a health functional food composition for the prevention or improvement of pulmonary fibrosis.

In the present invention, the details of the above taurodeoxycholic acid or pharmaceutically acceptable salt thereof are the same as described above, so a detailed explanation will refer to the above and only the specific composition of the health functional food composition will be described below.

Meanwhile, the present inventors have confirmed that, as a result of administering a pharmaceutically acceptable salt of taurodeoxycholic acid to a bleomycin-induced idiopathic pulmonary fibrosis animal model and a TGF-β-induced pulmonary fibrosis cell model, pulmonary fibrosis and the expression of factors related to pulmonary fibrosis were reduced. Therefore, the taurodeoxycholic acid or a pharmaceutically acceptable salt thereof can be used as an active ingredient in a health functional food composition for the prevention or improvement of pulmonary fibrosis.

The health functional food composition of the present invention can be manufactured in any one of the formulations selected from the group consisting of powder, granule, pill, tablet, capsule, candy, syrup, and beverage, but is not limited thereto.

The health functional food composition is not particularly limited as long as it can be ingested to prevent or improve the disease. When using the health functional food composition of the present invention as a food additive, it can be added as is or used together with other foods or food ingredients, and can be appropriately used according to conventional methods. The active ingredient can be appropriately used according to its intended use (prevention or improvement). Generally, when manufacturing food or beverages, it is added in an amount of 15 parts by weight or less, preferably 10 parts by weight or less, based on the health functional food composition of the present invention. However, in the case of long-term consumption for health regulation purposes, the amount may be less than the aforementioned range, and since there is no safety issue, the active ingredient can be used in an amount exceeding the aforementioned range. There is no particular limitation on the type of food. Examples of foods to which the health functional food composition can be added include meat, sausage, bread, chocolate, candies, snacks, cookies, pizza, ramen, other noodles, gum, dairy products including ice cream, various soups, beverages, tea drinks, alcoholic drinks, and vitamin complexes, as well as all health foods in the conventional sense. Additionally, the health functional food composition of the present invention can be manufactured as food, particularly functional food.

The functional food of the present invention includes components that are conventionally added during food manufacturing, such as proteins, carbohydrates, fats, nutrients, and seasonings. For example, when manufactured as a drink, it may include natural carbohydrates or flavoring agents as additional components, in addition to the active ingredient. The natural carbohydrates are preferably monosaccharides (e.g., glucose, fructose), disaccharides (e.g., maltose, sucrose), oligosaccharides, polysaccharides (e.g., dextrin, cyclodextrin), or sugar alcohols (e.g., xylitol, sorbitol, erythritol). The flavoring agents can be natural flavoring agents (e.g., thaumatin, stevia extract) or synthetic flavoring agents (e.g., saccharin, aspartame). In addition to the health functional food composition mentioned above, various nutrients, vitamins, electrolytes, flavor enhancers, colorants, pectic acid and its salts, alginic acid and its salts, organic acids, protective colloid thickening agents, pH regulators, stabilizers, preservatives, glycerin, alcohols, carbonating agents used in carbonated beverages and the like may also be included.

Hereinafter, the present invention will be described in detail with reference to examples.

However, the following examples are provided for illustrative purposes only, and the scope of the present invention is not limited to these examples.

### <Example 1> Preparation of Bleomycin-Induced Idiopathic Pulmonary Fibrosis Animal Model and Experimental Design

To investigate the efficacy of taurodeoxycholic acid (TDCA) in pulmonary fibrosis, a bleomycin-induced idiopathic pulmonary fibrosis animal model was established, and the sodium salt of TDCA, sodium taurodeoxycholate (hereinafter referred to as "HY209"), was administered to the animal model.

Specifically, 9-week-old C57BL/6 mice were purchased and allowed to adapt to their environment for one week before the experiment was conducted. The experimental animals were maintained under conditions of 22 ± 2°C, 55 ± 10% relative humidity, and a 12-hour light/dark cycle. Water was provided ad libitum, and sterilized feed was used, allowing free access to food.

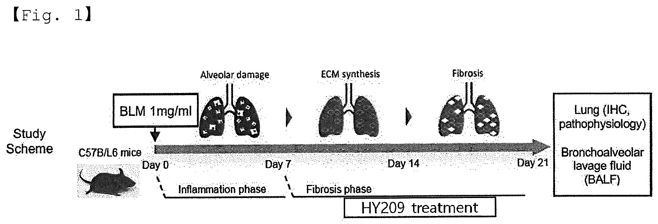

Next, as shown in Fig. 1, bleomycin (BLM, Enzo Life Sciences, Inc., Cas. No. 9041-93-4) was administered intranasally at a concentration of 1 mg/mL to the mice. Following this, the mice were divided into seven groups as indicated in Table 1, and samples were administered during the fibrosis phase (Day 7 to Day 21) after the inflammation phase (Day 0 to Day 7). The pulmonary fibrosis group was orally administered a vehicle (0.5% CMC (carboxymethylcellulose) solution) at a dose of 10 mL/kg twice daily, while the experimental group was orally administered HY209 formulated in a 0.5% CMC solution to achieve final concentrations of 10, 30, and 60 mg/kg, also twice daily. The comparison group (Nintedanib administration group) was orally administered Nintedanib, formulated in a vehicle (0.1% Natrosol; Hydroxyethylcellulose solution) to achieve a final concentration of 60 mg/kg, twice daily. The pulmonary fibrosis group (Natrosol administration group) for comparison with the Nintedanib administration group received 10 mL/kg of 0.1% Natrosol solution orally twice daily. The control group was administered saline instead of bleomycin and received a vehicle (0.5% CMC (carboxymethylcellulose) solution) at a dose of 10 mL/kg orally twice daily.

**[Table 1]**

| Group | Number /group | Challenge IN D0 | Administration (Day7-Day21) | Administration method | Administration Amounts | Administration frequency |
|---|---|---|---|---|---|---|
| Control group | 5 | saline | 0.5% CMC | | 10 mL/kg | BID |
| Pulmonary fibrosis group | 15 | bleomycin 1 mg/kg | 0.5% CMC | | 10 mL/kg | BID |
| HY209 10 mg/kg administration group | 15 | | HY209 | | 10 mg/kg | BID |
| HY209 30 mg/kg administration group | 15 | | HY209 | Oral | 30 mg/kg | BID |
| HY209 60 mg/kg administration group | 15 | | HY209 | | 60 mg/kg | BID |
| Nintendanib administration group | 15 | | Nintedanib | | *60* mg/kg | BID |
| Natrosol administration group | 15 | | 0.1% Natrosol | | 10 mL/kg | BID |

### <Example 2> Evaluation of Body Weight Changes Subsequent to Administration of HY209 in a Bleomycin-Induced Idiopathic Pulmonary Fibrosis Animal Model

The body weight changes of each mouse in Example 1 were assessed.

Specifically, the body weight of the mice in each group was measured daily throughout the course of the experiment.

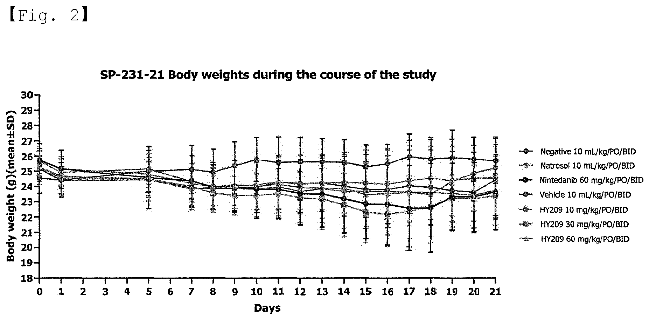

As a result, as shown in Fig. 2, there were no significant differences in body weight changes between the HY209 administration group and the control group.

### <Example 3> Evaluation of Lung Weight Changes Subsequent to Administration of HY209 in a Bleomycin-Induced Idiopathic Pulmonary Fibrosis Animal Model

The lung weight changes of each mouse in Example 1 were assessed.

Specifically, the mice in each group were sacrificed upon completion of the experiment, and their lungs were excised. The weights of the excised lungs were then measured.

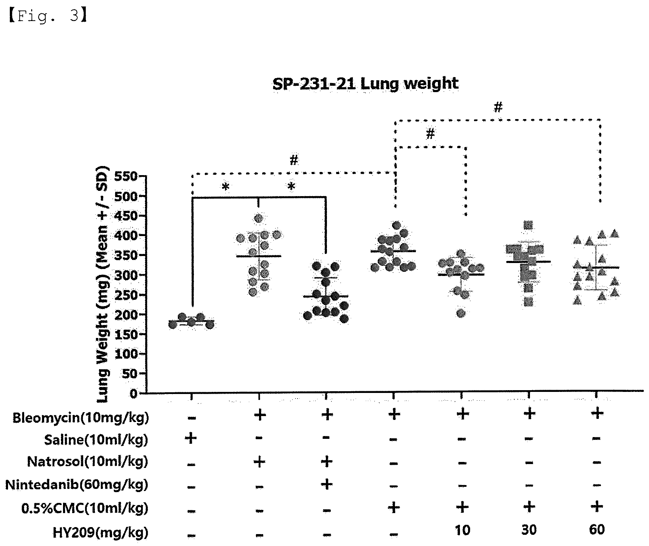

As a result, as shown in Fig. 3, the lung weights in the pulmonary fibrosis group and the Natrosol administration group were significantly increased compared to the control group due to bleomycin-induced idiopathic pulmonary fibrosis. In contrast, the Nintedanib administration group exhibited a significant decrease in lung weight compared to the Natrosol administration group. Furthermore, when compared to the pulmonary fibrosis group, the lung weights in the HY209 10 mg/kg administration group and the HY209 60 mg/kg administration group were statistically significantly decreased.

### <Example 4> Evaluation of Histopathological Changes Subsequent to Administration of HY209 in a Bleomycin-Induced Idiopathic Pulmonary Fibrosis Animal Model

The pulmonary fibrosis of each mouse in Example 1 was evaluated.

Specifically, the mice in each group were sacrificed upon completion of the experiment, and their lungs were excised. The excised lungs were embedded in paraffin for histopathological evaluation and stained according to the Crossman's Trichrome method (Gray P. The Microtomist's Formulary and Guide. Published by Robert E. Krieger Publishing Co.). The histological changes in the lungs were assessed using the Matsuse modification of the Ashcroft score, as presented in Table 2 (Ashcroft H et al. J Clin Pathol (1988) 41:467-70; Matsuse T et al. Eur Respir J (1999) 13:71-77).

**[Table 2]**

| Ashcroft score | Score |
|---|---|
| Normal lung (no fibrosis) | 1 |
| Minimal fibrotic thickening of alveolar or bronchial walls (network of fine collagen fibrils) | 2 |
| Moderate fibrotic thickening of walls without obvious damage to lung architecture | 3 |
| fibrosis with damage of pulmonary structrue(coarse fibrous bands or small fibrous masses, intra-alveolar collagen fibrils) | 4 |
| Large fibrous area with severe distortion of lung structure | 5 |

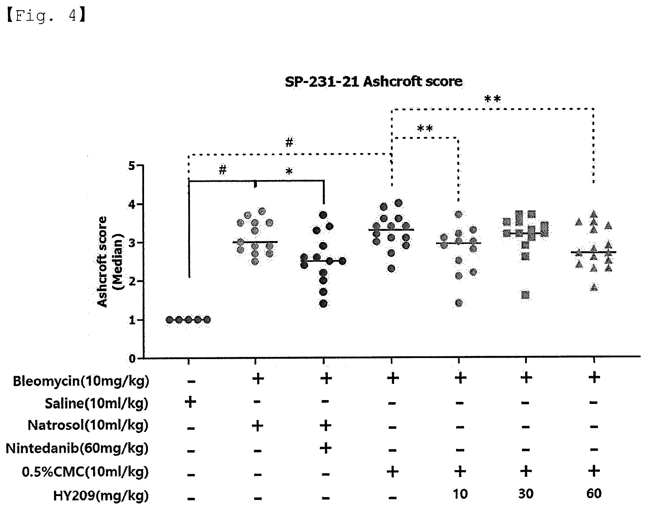

As a result, as shown in Fig. 4, pulmonary fibrosis was significantly increased in the pulmonary fibrosis group and the Natrosol administration group compared to the control group. In contrast, the Nintedanib administration group exhibited a significant reduction in pulmonary fibrosis compared to the Natrosol administration group. Additionally, when compared to the pulmonary fibrosis group, pulmonary fibrosis was statistically significantly decreased in the HY209 10 mg/kg administration group and the HY209 60 mg/kg administration group.

### <Example 5> Evaluation of Changes in the Expression of Pulmonary Fibrosis-Related Factors Subsequent to Administration of HY209 in a Bleomycin-Induced Idiopathic Pulmonary Fibrosis Animal Model.

The distribution of the expression of fibrosis-related factors in the lung tissue of each mouse in Example 1 was assessed.

Specifically, the mice in each group were sacrificed upon completion of the experiment, and their lungs were excised. The excised lungs were embedded in paraffin for histopathological evaluation, and sections were obtained and mounted onto slides. Subsequently, the expression distribution of the fibrosis-related factor α-SMA in the lung tissue was evaluated.

Immunohistochemical analysis of α-SMA was performed using polyclonal anti-α-SMA antibodies. Antigen retrieval was conducted using Target Retrieval Solution pH 6 (PT Link module, DAKO). The slides were reacted with primary rabbit polyclonal anti-α-SMA antibodies and subsequently reacted with the ImmPRESS detection kit (Autostainer Link48, DAKO). The de novo accumulation level of α-SMA was evaluated using digital image analysis software (Visiopharm^{®}). The results were expressed as the percentage (%) of the α-SMA area.

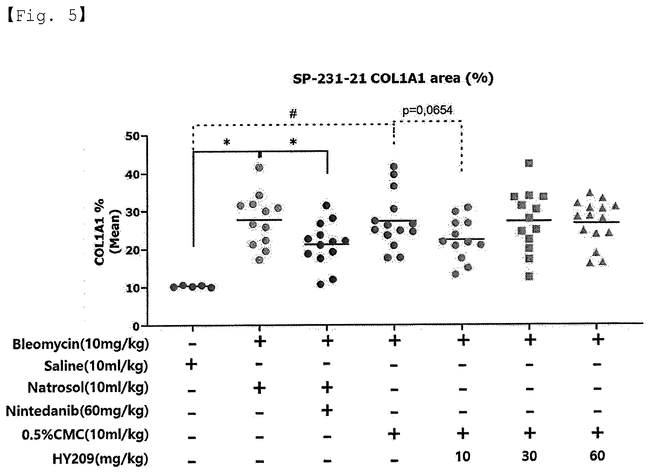

As a result, as shown in Fig. 5, the α-SMA area was significantly increased in the pulmonary fibrosis group and the Natrosol administration group compared to the control group. No significant difference was observed between the pulmonary fibrosis group and the Natrosol administration group.

In contrast, the Nintedanib administration group exhibited a significant reduction in the α-SMA area compared to both the pulmonary fibrosis group and the Natrosol administration group. Additionally, when compared to the pulmonary fibrosis group and the Natrosol administration group, the α-SMA area was significantly decreased in the HY209 10 mg/kg administration group and the HY209 60 mg/kg administration group.

### <Example 6> Comparison of the Inhibitory Effects of HY209 and TUDCA on the Expression of Fibrosis-Related Factors.

To compare the fibrosis-inhibitory effects of HY209 and TUDCA (tauroursodeoxycholic acid) in pulmonary fibrosis, the expression inhibition of fibrosis-related factors was measured by qPCR in the human lung fibroblast cell line MRC5, which was treated with TGF-β to induce fibrosis concurrently with the administration of HY209 and TUDCA.

Specifically, the human lung fibroblast cell line MRC5 was seeded at a density of 2 × 10^5 cells per well in a 6-well plate and incubated overnight to allow cell attachment. Subsequently, the medium was removed, and the cells were treated with TGF-β1 at a concentration of 5 ng/mL along with HY209 or TUDCA at concentrations of 0, 0.1, 1, and 10 µM, and cultured for 48 hours before collecting the cells. Following this, RNA was extracted from the cells using the RNeasy Mini Kit (QIAGEN), and cDNA was synthesized using Maxime^{™} RT PreMix (LiliF Diagnostic). qPCR measurements were conducted using Power SYBR^{™} Green PCR Master Mix (Thermo Scientific) in accordance with the manufacturer's instructions. Additionally, primers specific for Col1α1, α-SMA, and PAI-1 were utilized to assess their expression, and GAPDH was measured as the internal standard.

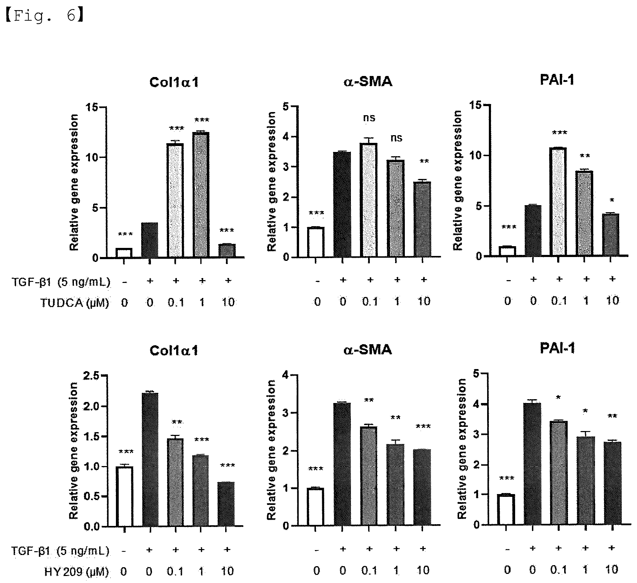

As a result, as shown in Fig. 6, HY209 exhibited inhibition of α-SMA and PAI-1 expression at concentrations of 0.1, 1, and 10 µM, whereas TUDCA did not demonstrate inhibitory effects at concentrations of 0.1 and 1 µM, showing inhibitory effects only at 10 µM. Furthermore, while HY209 reduced the expression of Col1α1 at concentrations of 0.1, 1, and 10 µM, TUDCA led to an increase in expression at concentrations of 0.1 and 1 µM.

The results indicate that the inhibitory effect of HY209 on the expression of fibrosis-related factors is significantly superior to that of TUDCA.

### <Example 7> Evaluation of the Anti-Inflammatory Efficacy of HY209 and TUDCA

To evaluate the anti-inflammatory efficacy of HY209 and TUDCA, the human monocytic cell line U937 was treated with PMA (phorbol 12-myristate 13-acetate) to induce differentiation into macrophages. Following this, inflammation was induced using BzATP (2'(3')-O-(4-Benzoylbenzoyl)adenosine 5'-triphosphate) or Pam2CSK4 (Pam2CysSerLys4), and the inhibitory effects of HY209 or TUDCA on the production of the inflammatory cytokines IL-1β and TNF-α were measured.

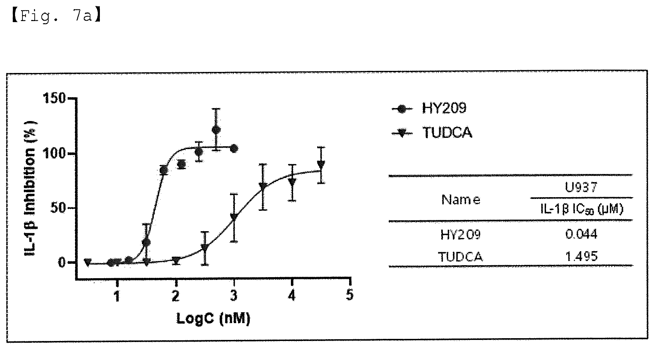

Specifically, the human monocytic cell line U937 was seeded at a density of 70,000 cells per well in a 96-well plate and cultured for 24 hours in RPMI1640 medium containing 50 nM PMA (phorbol 12-myristate 13-acetate) to induce differentiation into macrophages. The medium was then removed from each well, and HY209 or TUDCA was administered at varying concentrations (HY209: 1, 0.5, 0.25, 0.125, 0.0625, 0.0312, 0.0156, 0.0078 µM; TUDCA: 1000, 316, 100, 31.6, 10, 3.16, 0.316, 0.1, 0.0316, 0.01, 0.00316 µM) for 12 hours. Subsequently, BzATP (2'(3')-O-(4-Benzoylbenzoyl)adenosine 5'-triphosphate) was added to each well to achieve a final concentration of 300 µM, and the cells were incubated for an additional 12 hours. The supernatants were then collected, and IL-1β levels were measured using an IL-1β ELISA kit (R&D Systems) according to the manufacturer's instructions (Fig. 7a).

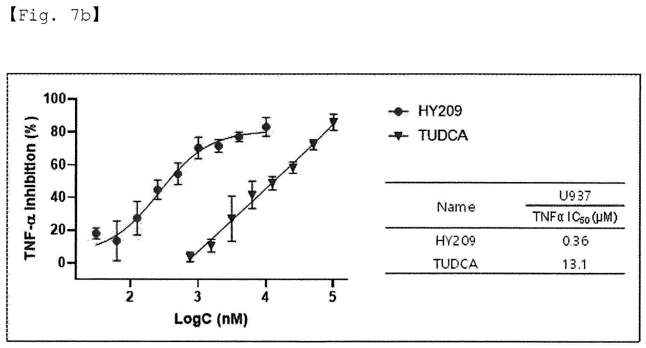

Additionally, the human monocytic cell line U937 was cultured at a density of 5 × 10^6 cells in a 100 mm dish containing RPMI1640 medium supplemented with 4 nM PMA (phorbol 12-myristate 13-acetate) for 5 hours to induce differentiation into macrophages. The differentiated macrophages were then seeded into a 96-well plate at a density of 50,000 cells per well and incubated for 24 hours to allow for rest. Following this, the medium was replaced with serum-free medium, and the cells were cultured for an additional 12 hours. The medium was then removed from each well, and the cells were treated with 50 ng/mL of Pam2CSK4 (Pam2CysSerLys4) along with HY209 or TUDCA at varying concentrations (HY209: 10, 4, 2, 1, 0.5, 0.25, 0.125, 0.0625, 0.03125 µM; TUDCA: 100, 50, 25, 12.5, 6.25, 3.125, 1.562, 0.76 µM) for 4 hours. The supernatants were then collected, and TNF-α levels were measured using a TNF-α ELISA kit (R&D Systems) according to the manufacturer's instructions (Fig. 7b).

The results, as illustrated in Fig. 7a and Fig. 7b, indicated that HY209 and TUDCA exhibited IC₅₀ values of 0.044 µM and 1.496 µM, respectively, for the inhibition of IL-1β production (Fig. 7a), while for TNF-α, the IC₅₀ values were 0.36 µM and 13.1 µM, respectively (Fig. 7b).

These results confirm that HY209 exhibits significantly superior anti-inflammatory efficacy compared to TUDCA, as evidenced by IC₅₀ values for IL-1β that are 34 times lower and for TNF-α that are 36 times lower than those of TUDCA.

### [Industrial Applicability]

In the present invention, it has been confirmed that a pharmaceutically acceptable salt of taurodeoxycholic acid reduces pulmonary fibrosis and the expression of pulmonary fibrosis-related factors in both an idiopathic pulmonary fibrosis animal model and a pulmonary fibrosis cell model. Therefore, taurodeoxycholic acid or a pharmaceutically acceptable salt thereof of the present invention can be utilized as an active ingredient in a composition for the prevention or treatment of pulmonary fibrosis.

## Claims

1. A pharmaceutical composition for use in the prevention or treatment of pulmonary fibrosis, comprising taurodeoxycholic acid or a pharmaceutically acceptable salt thereof as an active ingredient.

2. The pharmaceutical composition for use in the prevention or treatment of pulmonary fibrosis according to claim 1, wherein the taurodeoxycholic acid is a compound represented by the following [Chemical Formula 2]:

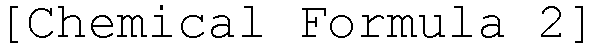

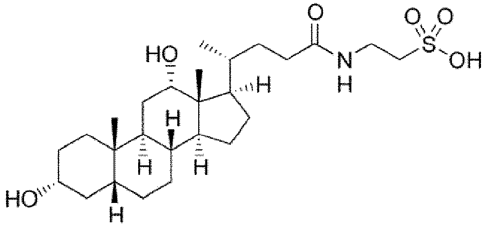

3. The pharmaceutical composition for use in the prevention or treatment of pulmonary fibrosis according to claim 1, wherein the pharmaceutically acceptable salt is a sodium salt.

4. The pharmaceutical composition for use in the prevention or treatment of pulmonary fibrosis according to claim 1, wherein the composition reduces the expression of factors related to pulmonary fibrosis.

5. The pharmaceutical composition for use in the prevention or treatment of pulmonary fibrosis according to claim 4, wherein the factors related to pulmonary fibrosis are COL1α1, α-SMA, or PAI-1.

6. The pharmaceutical composition for use in the prevention or treatment of pulmonary fibrosis according to claim 1, wherein the pulmonary fibrosis is selected from the group consisting of idiopathic pulmonary fibrosis, nonspecific interstitial pneumonia, acute interstitial pneumonia, cryptogenic organizing pneumonia, respiratory bronchiolitis-associated interstitial lung disease, desquamative interstitial pneumonia, lymphoid interstitial pneumonia, interstitial pulmonary fibrosis, and diffuse pulmonary fibrosis.

7. The pharmaceutical composition for use in the prevention or treatment of pulmonary fibrosis according to claim 1, further comprising one or more pharmaceutically acceptable carriers.

8. A health functional food composition for use in the prevention or improvement of pulmonary fibrosis, comprising taurodeoxycholic acid or a pharmaceutically acceptable salt thereof as an active ingredient.

9. A food composition for use in the prevention or improvement of pulmonary fibrosis, comprising taurodeoxycholic acid or a pharmaceutically acceptable salt thereof as an active ingredient.

## Patentansprüche

1. Eine pharmazeutische Zusammensetzung zur Verwendung bei der Vorbeugung oder Behandlung von Lungenfibrose, umfassend Taurodeoxycholsäure oder ein pharmazeutisch verträgliches Salz davon als Wirkstoff.

2. Die pharmazeutische Zusammensetzung zur Verwendung bei der Vorbeugung oder Behandlung von Lungenfibrose gemäß Anspruch 1, wobei die Taurodeoxycholsäure eine Verbindung ist, dargestellt durch die folgende [Chemische Formel 2]:

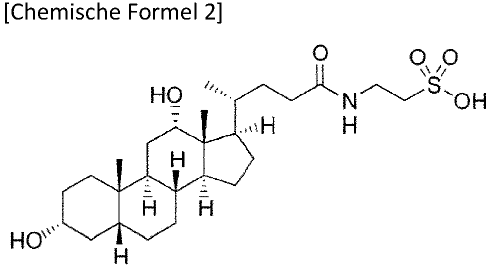

3. Die pharmazeutische Zusammensetzung zur Verwendung bei der Vorbeugung oder Behandlung von Lungenfibrose gemäß Anspruch 1, wobei das pharmazeutisch verträgliche Salz ein Natriumsalz ist.

4. Die pharmazeutische Zusammensetzung zur Verwendung bei der Vorbeugung oder Behandlung von Lungenfibrose gemäß Anspruch 1, wobei die Zusammensetzung die Expression von Faktoren, die mit Lungenfibrose in Zusammenhang stehen, verringert.

5. Die pharmazeutische Zusammensetzung zur Verwendung bei der Vorbeugung oder Behandlung von Lungenfibrose gemäß Anspruch 4, wobei die Faktoren, die mit Lungenfibrose in Zusammenhang stehen, COL1α1, α-SMA oder PAI-1 sind.

6. Die pharmazeutische Zusammensetzung zur Verwendung bei der Vorbeugung oder Behandlung von Lungenfibrose gemäß Anspruch 1, wobei die Lungenfibrose ausgewählt ist aus der Gruppe bestehend aus idiopathischer Lungenfibrose, unspezifischer interstitieller Pneumonie, akuter interstitieller Pneumonie, kryptogener organisierender Pneumonie, respiratorischer bronchiolitis-assoziierter interstitieller Lungenerkrankung, desquamativer interstitieller Pneumonie, lymphoider interstitieller Pneumonie, interstitieller Lungenfibrose und diffuser Lungenfibrose.

7. Die pharmazeutische Zusammensetzung zur Verwendung bei der Vorbeugung oder Behandlung von Lungenfibrose gemäß Anspruch 1, die ferner einen oder mehrere pharmazeutisch verträgliche Trägerstoffe enthält.

8. Eine gesundheitsfunktionelle Lebensmittelzusammensetzung zur Verwendung bei der Vorbeugung oder Besserung von Lungenfibrose, umfassend Taurodeoxycholsäure oder ein pharmazeutisch verträgliches Salz davon als Wirkstoff.

9. Eine Nahrungsmittelzusammensetzung zur Verwendung bei der Vorbeugung oder Besserung von Lungenfibrose, die Taurodeoxycholsäure oder ein pharmazeutisch verträgliches Salz davon als Wirkstoff enthält.

## Revendications

1. Composition pharmaceutique pour son utilisation dans la prévention ou le traitement d'une fibrose pulmonaire, comprenant, en tant que principe actif, de l'acide taurodésoxycholique ou un sel pharmaceutiquement acceptable de celui-ci.

2. Composition pharmaceutique pour son utilisation dans la prévention ou le traitement d'une fibrose pulmonaire selon la revendication 1, dans laquelle l'acide taurodésoxycholique est un composé représenté par la [formule chimique 2] suivante :

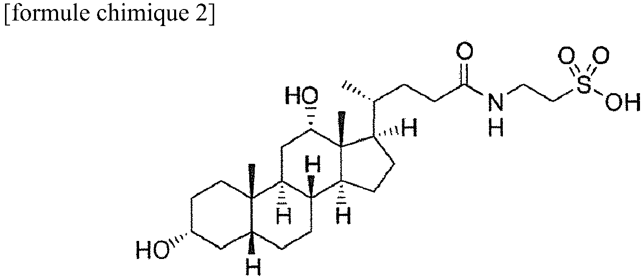

3. Composition pharmaceutique pour son utilisation dans la prévention ou le traitement d'une fibrose pulmonaire selon la revendication 1, dans laquelle le sel pharmaceutiquement acceptable est un sel de sodium.

4. Composition pharmaceutique pour son utilisation dans la prévention ou le traitement d'une fibrose pulmonaire selon la revendication 1, dans laquelle la composition réduit l'expression de facteurs associés à une fibrose pulmonaire.

5. Composition pharmaceutique pour son utilisation dans la prévention ou le traitement d'une fibrose pulmonaire selon la revendication 4, dans laquelle les facteurs associés à une fibrose pulmonaire sont COL1α1, α-SMA, ou PAI-1.

6. Composition pharmaceutique pour son utilisation dans la prévention ou le traitement d'une fibrose pulmonaire selon la revendication 1, dans laquelle la fibrose pulmonaire est choisie dans le groupe constitué par une fibrose pulmonaire idiopathique, une pneumopathie interstitielle non spécifique, une pneumopathie interstitielle aiguë, une pneumopathie organisée cryptogénique, une maladie pulmonaire interstitielle associée à une bronchiolite respiratoire, une pneumopathie interstitielle desquamative, une pneumopathie interstitielle lymphoïde, une fibrose pulmonaire interstitielle, et une fibrose pulmonaire diffuse.

7. Composition pharmaceutique pour son utilisation dans la prévention ou le traitement d'une fibrose pulmonaire selon la revendication 1, comprenant en outre un ou plusieurs véhicules pharmaceutiquement acceptables.

8. Composition d'aliment fonctionnel pour la santé pour son utilisation dans la prévention ou l'amélioration d'une fibrose pulmonaire, comprenant, en tant que principe actif, de l'acide taurodésoxycholique ou un sel pharmaceutiquement acceptable de celui-ci.

9. Composition alimentaire pour son utilisation dans la prévention ou l'amélioration d'une fibrose pulmonaire, comprenant, en tant que principe actif, de l'acide taurodésoxycholique ou un sel pharmaceutiquement acceptable de celui-ci.
